# FASCICULE DE BREVET EUROPEEN

(11) **EP 4 588 346 B1**
(45) Date de publication et mention de la délivrance du brevet: **08.07.2026**
(21) Numéro de dépôt: 25152606.7
(22) Date de dépôt: 17.01.2025
(51) Int. Cl.: A01K 67/33, A23K 50/75, C05F 17/05, C05F 17/90

(54) **MILIEU NUTRITIF ET PROCÉDÉ POUR LA PRODUCTION DE LOMBRICS DESTINÉS À L'ALIMENTATION ANIMALE**
NÄHRMEDIUM UND VERFAHREN ZUR HERSTELLUNG VON LUMBRICS FÜR TIERFUTTER
NUTRIENT MEDIUM AND PROCESS FOR THE PRODUCTION OF ANIMAL FEED EARTHWORMS

(30) Priorité: 17.01.2024 FR 2400467
(43) Date de publication de la demande: 23.07.2025
(73) Titulaire: Vers les Poules, 85710 Bois-de-Céné (FR)
(72) Inventeur: RICHARD, Laurent, 85710 BOIS-DE-CENE (FR)
(74) Mandataire: Jacobacci & Partners France

(56) Documents cités:
- CN-A- 104 261 921
- CN-A- 107 382 391
- CN-A- 113 057 144
- CN-A- 117 084 219
- FR-A1- 3 114 730
- ASSANDRI DAVIDE ET AL: "Suitability of Composting Process for the Disposal and Valorization of Brewer's Spent Grain", AGRICULTURE, vol. 11, no. 1, 22 December 2020 (2020-12-22), pages 2, XP093198102, ISSN: 2077-0472, DOI: 10.3390/agriculture11010002

## Description

### Domaine technique de l'invention

La présente invention concerne le domaine général de la production de lombrics (ou vers de terre).

Elle concerne plus particulièrement un milieu nutritif et un procédé pour la production de lombrics destinés à l'alimentation animale (volailles, porcs et poissons notamment).

### Etat de la technique

Les lombrics peuvent participer de manière efficace au régime alimentaire de divers animaux tels que les volailles (poules pondeuses, poulets à viande, etc.), les porcs ou encore les poissons, en remplacement du soja, farine de poisson et acides aminés de synthèse.

Notamment, le document WO 2019/234653 décrit un procédé et une installation pour la production de vers (lombrics) et de compost de vers (lombricompost). Cependant la technologie proposée ne permet pas d'assurer un développement optimal des lombrics.

CN 104 261 921 A divulgue le traitement circulaire de la bouse de vache par l'intermédiaire d'un bassin de lombricompostage.

CN 117 084 219 A divulgue l'utilisation de drêches de brasserie pour nourrir des larves de Ténébrion meunier.

Pour développer une filière rentable, la production de vers de terre destinés à l'alimentation d'animaux doit être rapide et respecter les normes en vigueur.

Il est donc nécessaire de mettre en place des conditions de développement optimal pour les lombrics, et assurer leur alimentation efficace par des matières premières disponibles localement.

### Présentation de l'invention

Pour cela, la présente invention propose un milieu nutritif de base pour la production de lombrics destinés à l'alimentation animale,
ledit milieu nutritif de base étant caractérisé en ce qu'il comprend :
(a) un rapport C/N compris entre 25 et 45,
(b) une proportion de lombricompost produit par des lombrics, en forme de milieu nutritif final issu d'une étape de séparation entre des lombrics et leur milieu nutritif,
(c) un mélange de différents composants, parmi lesquels une proportion majoritaire en poids de drêches de brasserie, hors ajout de lombricompost.

D'autres caractéristiques non limitatives et avantageuses du milieu nutritif conforme à l'invention, prises individuellement ou selon toutes les combinaisons techniquement possibles, sont les suivantes :
- Le milieu nutritif de base comprend un mélange d'une certaine proportion de composants choisis parmi les algues, le foin de légumineuses, les pommes de terre, les cosses de Sarrazin, les ballots de champignonnière (co-produits de champignonnière), la paille et le carton, en plus des drêches de brasserie.
- Le milieu nutritif de base comprend deux, trois ou quatre des composants, choisis parmi les drêches de brasserie, les algues, le foin de légumineuses, les pommes de terre, les cosses de Sarrazin, les ballots de champignonnières, la paille et le carton.
- Le milieu nutritif de base comprend des drêches de brasserie, des pommes de terre, des cosses de Sarrazin, et des ballots de champignonnière.
- Le milieu nutritif de base comprend un mélange de différents composants réduits en petites particules, dont la taille est comprise entre 0 et 5 millimètres.
- La proportion de lombricompost qui est ajoutée au mélange de composants est comprise entre 10 et 50% en poids.

L'invention concerne également un procédé de production de lombrics destinés à l'alimentation animale, utilisant le milieu nutritif de base défini ci-dessus, lequel procédé consiste :
- à produire un milieu de culture en forme de lombricompost par des lombrics,
- à préparer un milieu nutritif de base tel que défini ci-dessus,
- à incorporer une quantité de lombrics dans ledit milieu nutritif de base,
- à laisser les lombrics se développer pendant une période de croissance déterminée, cela tout en contrôlant le taux d'hygrométrie du milieu nutritif de telle sorte que ce taux d'hygrométrie soit compris entre 75 et 90%,
- en fin de période de croissance, à séparer les lombrics et leur milieu nutritif,
- éventuellement à mettre en œuvre au moins une opération de transformation des lombrics pour obtenir un produit transformé à base de lombric,
- à utiliser les lombrics, ou le produit transformé à base de lombric, pour l'alimentation animale.

De préférence, la période de croissance mentionnée ci-dessus est comprise entre deux et quatre mois.

Un tel procédé permet d'optimiser la croissance et la multiplication des lombrics.

### Description détaillée de l'invention

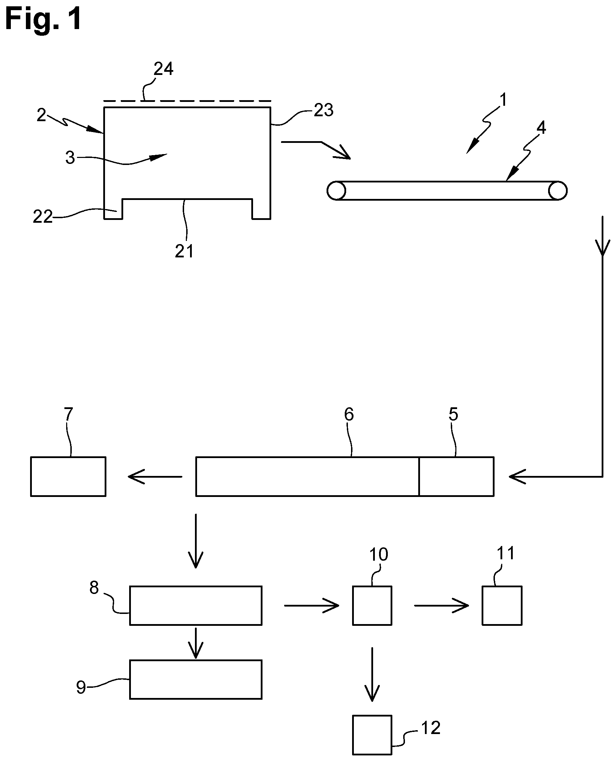

D'autres caractéristiques de l'invention ressortent de la description suivante effectuée en référence au dessin annexé de la figure 1 qui est une vue générale et schématique d'une installation pour la production de lombrics destinés à l'alimentation animale.

### Description générale du procédé.

Le procédé de production de lombrics destinés à l'alimentation animale conforme à l'invention consiste :
(a) à préparer un milieu nutritif de base comprenant un mélange de différents composants adaptés pour obtenir un mélange dont le rapport C/N est compris entre 25 et 45, et à ajouter dans ce mélange de composants une proportion d'un milieu de culture de lombrics issu d'une production préalable,
(b) à incorporer dans le milieu nutritif de base une quantité de lombrics,
(c) à laisser les lombrics se développer pendant une période de croissance déterminée (avantageusement comprise entre deux et quatre mois), cela tout en contrôlant le taux d'hygrométrie du milieu nutritif de base de telle sorte que ce taux d'hygrométrie soit compris entre 75 et 90%,
(d) en fin de période de croissance, à séparer les lombrics et leur milieu nutritif,
(e) éventuellement, à mettre en œuvre au moins une opération de transformation des lombrics pour obtenir un produit transformé à base de lombric, et
(f) à utiliser les lombrics, ou le produit transformé à base de lombric, pour l'alimentation animale.

L'hygrométrie du milieu de culture est par exemple contrôlée une fois par semaine et, si besoin, ajustée entre 75 et 90% par apport d'eau.

Pour une croissance optimale, la température du milieu de culture est située entre 10 et 30°C, et son pH entre 6 et 8.

De préférence, dans le milieu nutritif de base, les composants sont réduits en petites particules dont la taille est inférieure à 5 millimètres. Cette opération est réalisée par broyage des composants dans un broyeur adapté pour obtenir une taille de particules comprise entre 0 et 5 millimètres.

L'incorporation des lombrics dans le milieu nutritif de base (étape (b)) est de préférence réalisée 14 à 22 jours après la préparation dudit milieu nutritif de base.

D'autre part, toujours de manière préférée, le milieu de culture mentionné à l'étape (a), qui est ajouté au mélange de composants, consiste en du milieu de culture (ou milieu nutritif) obtenu en fin de période de croissance, lors de la séparation entre les lombrics et leur milieu nutritif (étape (d)) d'une production précédente de lombrics.

Cette proportion de milieu nutritif ajouté est avantageusement comprise entre 10 et 50% du poids du mélange de composants.

### Description de l'installation

Une installation pour la mise en œuvre du procédé en vue de la production de lombrics est schématisée sur la figure 1.

Cette installation 1 est de préférence aménagée dans un local fermé permettant de conserver une température entre +5°C et +30°C.

L'installation 1 correspondante comprend au moins un contenant de croissance 2 (et de préférence plusieurs contenants de croissance 2), dont le volume intérieur 3 est destiné à recevoir un milieu nutritif de base et des lombrics, et qui est adapté pour la croissance des lombrics dans le milieu nutritif pendant la période de croissance.

Le contenant de croissance 2 consiste de préférence en une boite ou caisse de forme générale parallélépipédique, réalisée en bois, comprenant un élément de fond 21 monté sur un piètement de surélévation 22 et qui est bordé par des parois latérales 23.

Son ouverture supérieure peut recevoir un élément de couvercle amovible 24 muni de perforations.

Le contenant de croissance 2 peut avoir un volume de l'ordre de 1m³, avec des dimensions de 1m x 1m x 1m.

L'installation 1 pour la mise en œuvre du procédé selon l'invention comprend encore un système en forme de convoyeur 4, par exemple un convoyeur du type à rouleaux, adapté pour la prise en charge du ou des contenants de croissance 2, et pour les transférer jusqu'à un dispositif déverseur 5 chargé d'alimenter un dispositif de séparation 6 conçu pour séparer les lombrics et le milieu nutritif (consistant alors en un produit que l'on peut dénommer « lombricompost »), après la fin de la période de croissance.

Le dispositif déverseur 5 peut consister en un gerbeur de type à doigts.

De son côté, le dispositif de séparation 6 peut consister en un appareil de tamisage du type tamis rotatif TROMMEL

Le milieu nutritif (lombricompost) est réceptionné par un convoyeur sans fin 7 pour son transfert vers une zone de stockage, en vue sa valorisation en particulier comme fertilisant, ou en vue de son incorporation dans le milieu nutritif de base d'une production ultérieure de lombrics.

De leur côté, les lombrics sont réceptionnés par un convoyeur sans fin 8 qui les transfère :
- en direction d'un contenant de réception 9, pour leur conditionnement vivants à utiliser tels quels pour l'alimentation animale, ou
- en direction d'une installation de transformation 10, pour obtenir un produit transformé à base de lombric.

Par exemple, l'installation de transformation 10 peut comprendre un matériel de type extrudeuse pour l'obtention d'une phase aqueuse/huileuse 11, et d'une phase solide 12.

### Le milieu nutritif de base.

Le milieu nutritif de base comprend un mélange d'une certaine proportion de composants qui peuvent être choisis notamment parmi les algues, le foin de légumineuses, la paille, les drêches de brasserie, le carton, la pomme de terre, les cosses de Sarrazin et les ballots de champignonnière (co-produits de champignonnière).

De préférence le milieu nutritif de base comprend au moins l'un des composants précités, encore de préférence, il comprend deux, trois ou quatre de ces composants ; il peut aussi comprendre l'ensemble de ces composants.

Les composants sont choisis en fonction de leurs facilité d'approvisionnement, et également en fonction du rapport C/N recherché au final, à savoir un rapport C/N compris entre 25 et 45, tenant compte des valeurs de base classiques mentionnées dans le tableau 1 ci-dessous.

**Tableau 1**

| | C/N |
|---|---|
| ALGUE | 17 |
| FOIN DE LEGUMINEUSE | 10 |
| PAILLE | 65 |
| DRECHES DE BRASSERIE | 10 |
| CARTON | 150 |
| COQUILLES D'OEUF | 25 |
| POMMES DE TERRE | 25 |
| COSSES DE SARRAZIN | 180 |
| BALLOTS DE CHAMPIGNONNIERE | 200 |

| | |
|---|---|
| Où C/N correspond au ratio Carbone sur Azote connu de la matière première (MP) mentionnée. | |

On peut par exemples utiliser un mélange de composants correspondant aux exemples 1 à 5 suivants :

### Exemple 1

| | % D'INCORPORATION | C/N MP | C/N MELANGE |
|---|---|---|---|
| ALGUE | 15 | 17 | 2,55 |
| FOIN DE LEGUMINEUSE | | 10 | 0 |
| PAILLE | 40 | 65 | 26 |
| DRECHES BRASSERIE | 35 | 10 | 3,5 |
| CARTON | 5 | 150 | 7,5 |
| COQUILLE D'ŒUF | 5 | 25 | 1,25 |
| | 100 | | 40,8 |

### Exemple 2

| | % D'INCORPORATION | C/N MP | C/N MELANGE |
|---|---|---|---|
| ALGUE | | 17 | 0 |
| FOIN DE LEGUMINEUSE | 30 | 10 | 3 |
| PAILLE | 25 | 65 | 16,25 |
| DRECHES BRASSERIE | 35 | 10 | 3,5 |
| CARTON | 5 | 150 | 7,5 |
| COQUILLE D'ŒUF | 5 | 25 | 1,25 |
| | 100 | | 31,5 |

### Exemple 3

| | % D'INCORPORATION | C/N MP | C/N MELANGE |
|---|---|---|---|
| ALGUE | | 17 | 0 |
| FOIN DE LEGUMINEUSE | 35 | 10 | 3,5 |
| PAILLE | | 65 | 0 |
| DRECHES BRASSERIE | 40 | 10 | 4 |
| CARTON | 20 | 150 | 30 |
| COQUILLE D'ŒUF | 5 | 25 | 1,25 |
| | 100 | | 38,75 |

### Exemple 4

| | % D'INCORPORATION | C/N MP | C/N MELANGE |
|---|---|---|---|
| ALGUE | 15 | 17 | 2,55 |
| FOIN DE LEGUMINEUSE | 20 | 10 | 2 |
| PAILLE | | 65 | 0 |
| DRECHES BRASSERIE | 40 | 10 | 4 |
| CARTON | 15 | 150 | 22,5 |
| COQUILLE D'ŒUF | 10 | 25 | 2,5 |
| | 100 | | 33,55 |

### Exemple 5

| | % D'INCORPORATION | C/N MP | C/N MELANGE |
|---|---|---|---|
| COSSES DE SARRAZIN | 3 | 180 | 5,4 |
| POMME DE TERRE | 15 | 25 | 3,75 |
| DRECHES BRASSERIE | 80 | 20 | 16 |
| BALLOTS DE CHAMPIGNONNIERE | 2 | 200 | 4 |
| | 100 | | 29,15 |

### Exemple de procédé de production de lombrics :

### - Phase 1 : Mélange des matières premières

Après leur préparation par broyage, on réalise le mélange choisi des matières premières et on laisse le mélange en l'état quelques jours.

Le mélange de matières premières obtenu doit être le plus fin possible pour faciliter la dégradation par les lombrics (la taille des particules des composants est comprises entre 0 et 10 millimètres, et de préférence comprise entre 0 et 5 millimètres). Il doit également être homogène ou relativement homogène.

Cette phase permet la montée en température du mélange (25 - 30°C) et se passe en anaérobie. Elle est importante pour optimiser la multiplication des lombrics et leur croissance en permettant durant 14 à 22 jours (par exemple 18 jours) le développement des bactéries et des champignons.

### - Phase 2 : remplissage des contenants de croissance avec le milieu nutritif de base maturé et les lombrics

Dans chaque contenant en bois on incorpore 1 tonne de mélange de matières premières maturé et 15 Kg de lombrics. Pour faciliter leur implantation dans le nouveau milieu de croissance, on complète le mélange de matières premières par une quantité d'un milieu nutritif final de fin de croissance issu d'une production précédente. Cela permet d'optimiser la croissance et d'éviter une mortalité trop importante lors de cette manipulation.

Par exemple on ajoute 15 à 20 Kg du milieu nutritif final de fin de croissance des lombrics, que l'on mélange avec le mélange de matières premières maturé.

Le mélange des matières premières avec le milieu nutritif final est réalisé juste avant l'incorporation dans le contenant et ensuite les lombrics sont introduits dans ce mélange.

En fonction des débouchés de valorisation du lombricompost, on peut faire le choix d'incorporer au milieu nutritif de base jusqu'à 50% en poids de milieu nutritif final, pour permettre une croissance plus rapide des lombrics.

Il conviendra d'arroser les contenants de croissance pour obtenir une humidité de 75 à 90% (80% en moyenne), et de recouvrir ces contenants pour limiter l'évaporation. L'eau utilisée pour humidifier les contenants peut être, en cas d'utilisation d'algues dans le milieu nutritif de base, celle qui a servi à les laver car elle contient des nutriments utilisables par les lombrics pour leur croissance.

Cette période de croissance peut durer environ 2 à 4 mois ; elle dure en moyenne environ 3 mois.

### - Phase 3 : la séparation des lombrics et du milieu nutritif final (lombricompost).

Dans un schéma industriel, les contenants de croissance sont posés sur un convoyeur à rouleau 4, qui est simple à nettoyer, et qui permet de les déplacer jusqu'au dispositif déverseur 5.

Le dispositif déverseur 5 permet de vider complétement les contenants de croissance sur un tapis convoyeur agencé pour amener le mélange milieu/lombrics au tamis 6 en vue de séparer les deux produits.

Après séparation, le lombricompost est stocké en vue de sa commercialisation comme fertilisant ou de son utilisation pour compléter le milieu nutritif de base.

### Utilisation des lombrics

Les lombrics produits peuvent être utilisés en l'état, vivants, pour l'alimentation animale.

Par exemple :
- Pour des poules pondeuses, une ration alimentaire de lombrics peut être de l'ordre de 8% de l'alimentation globale, soit pour une poule consommant 120 grammes d'aliment, 10 grammes de lombrics/jour.
- Pour des faisans, une ration alimentaire de lombrics peut être de l'ordre de 8% de l'alimentation globale, soit pour un faisan consommant 50 grammes d'aliment, 4 grammes de lombrics/jour.

Les lombrics peuvent également subir un processus de transformation avant leur utilisation comme aliment.

Les tests de production de lombrics donnent, pour un mélange initial de 1000 kg de milieu nutritif de base et 15 kg de lombrics, une production au bout de 3 mois de 350 kg de lombrics.

## Revendications

1. Milieu nutritif de base pour la production de lombrics destinés à l'alimentation animale, ledit milieu nutritif de base comprenant un rapport C/N compris entre 25 et 45, une proportion de lombricompost produit par des lombrics, en forme de milieu nutritif final issu d'une étape de séparation entre des lombrics et leur milieu nutritif, **caractérisé en ce qu'**il comprend un mélange de différents composants, parmi lesquels une proportion majoritaire en poids de drêches de brasserie, hors ajout de lombricompost.

2. Milieu nutritif de base selon la revendication 1, **caractérisé en ce qu'**il comprend un mélange d'une certaine proportion de composants choisis parmi les algues, le foin de légumineuses, les pommes de terre, les cosses de Sarrazin, les ballots de champignonnières, la paille et le carton, en plus des drêches de brasserie.

3. Milieu nutritif de base selon la revendication 2, **caractérisé en ce qu'**il comprend deux, trois ou quatre des composants, choisis parmi les drêches de brasserie, les algues, le foin de légumineuses, les pommes de terre, les cosses de Sarrazin, les ballots de champignonnières, la paille et le carton.

4. Milieu nutritif de base selon l'une quelconque des revendications 1 à 3, **caractérisé en ce qu'**il comprend des drêches de brasserie, de la pomme de terre, des cosses de Sarrazin et des ballots de champignonnières.

5. Milieu nutritif de base selon l'une quelconque des revendications 1 à 4, **caractérisé en ce qu'**il comprend un mélange de différents composants réduits en petites particules, dont la taille est comprise entre 0 et 5 millimètres.

6. Milieu nutritif de base selon l'une quelconque des revendications 1 à 5, **caractérisé en ce que** la proportion de lombricompost qui est ajoutée au mélange de composants est comprise entre 10 et 50% en poids.

7. Procédé de production de lombrics destinés à l'alimentation animale, utilisant le mélange nutritif de base selon l'une quelconque des revendications 1 à 6, lequel procédé consiste :
- à produire un milieu de culture en forme de lombricompost par des lombrics,
- à préparer un milieu nutritif de base selon l'une quelconque des revendications 1 à 6,
- à incorporer une quantité de lombrics dans ledit milieu nutritif de base,
- à laisser les lombrics se développer pendant une période de croissance déterminée, cela tout en contrôlant le taux d'hygrométrie du milieu nutritif de telle sorte que ce taux d'hygrométrie soit compris entre 75 et 90%,
- en fin de période de croissance, à séparer les lombrics et leur milieu nutritif,
- éventuellement à mettre en œuvre au moins une opération de transformation des lombrics pour obtenir un produit transformé à base de lombric,
- à utiliser les lombrics, ou le produit transformé à base de lombric, pour l'alimentation animale.

8. Procédé de production de lombrics selon la revendication 7, **caractérisé en ce qu'**il consiste à laisser les lombrics se développer pendant une période de croissance comprise entre deux et quatre mois.

## Patentansprüche

1. Basisnährmedium für die Produktion von Würmern, die als Tierfutter bestimmt sind, wobei das Basisnährmedium ein C/N-Verhältnis zwischen 25 und 45, einen Anteil an von Würmern erzeugtem Wurmkompost, in Form eines endgültigen Nährmediums aus einem Trennungsschritt zwischen Würmern und ihrem Nährmedium umfasst, **dadurch gekennzeichnet, dass** es eine Mischung verschiedener Komponenten umfasst, darunter einen überwiegenden Gewichtsanteil an Braugersten, ohne Zugabe von Wurmkompost.

2. Basisnährmedium nach Anspruch 1, **dadurch gekennzeichnet, dass** es eine Mischung eines gewissen Anteils von Komponenten umfasst, die aus Algen, Heu von Hülsenfrüchten, Kartoffeln, Buchweizenschalen, Pilzballen, Stroh und Pappkarton sowie Braukörnern ausgewählt sind.

3. Basisnährmedium nach Anspruch 2, **dadurch gekennzeichnet, dass** es zwei, drei oder vier der Komponenten umfasst, die aus Braukörnern, Algen, Heu von Hülsenfrüchten, Kartoffeln, Buchweizenschalen, Pilzballen, Stroh und Pappkarton ausgewählt sind.

4. Basisnährmedium nach einem der Ansprüche 1 bis 3, **dadurch gekennzeichnet, dass** es Braukörner, Kartoffeln, Buchweizenschalen und Pilzballen umfasst.

5. Basisnährmedium nach einem der Ansprüche 1 bis 4, **dadurch gekennzeichnet, dass** es eine Mischung verschiedener zu kleinen Partikeln reduzierter Komponenten umfasst, deren Größe zwischen 0 und 5 Millimetern beträgt.

6. Basisnährmedium nach einem der Ansprüche 1 bis 5, **dadurch gekennzeichnet, dass** der Anteil an Wurmkompost, der dem Komponentengemisch hinzugefügt wird, zwischen 10 und 50 Gew.-% beträgt.

7. Verfahren zur Herstellung von Würmern als Tierfutter unter Verwendung der Nährstoffbasismischung nach einem der Ansprüche 1 bis 6, wobei das Verfahren besteht aus:
- dem Erzeugen eines Kulturmediums in Form von Wurmkompost durch Würmer,
- dem Zubereiten eines Basisnährmediums nach einem der Ansprüche 1 bis 6,
- dem Einbringen einer Menge von Würmern in das Nährmedium,
- dem Wachsenlassen der Würmer während eines bestimmten Wachstumszeitraums und dabei das Kontrollieren des Feuchtigkeitsgehalts des Nährmediums, so dass dieser Feuchtigkeitsgehalt zwischen 75 und 90 % beträgt,
- dem Trennen der Würmer und ihr Nährmedium am Ende der Wachstumsphase,
- gegebenenfalls dem Durchführen mindestens eines Verarbeitungsvorgangs der Würmer, um ein verarbeitetes Produkt auf Wurmbasis zu erhalten,
- dem Verwenden der Würmer oder des verarbeiteten Produkts auf Wurmbasis als Tierfutter.

8. Verfahren zur Herstellung von Würmern nach Anspruch 7, **dadurch gekennzeichnet, dass** es darin besteht, die Würmer über einen Wachstumszeitraum von zwei bis vier Monaten wachsen zu lassen.

## Claims

1. A basic nutrient medium for the production of earthworms intended for animal feed, said basic nutrient medium comprising a C/N ratio between 25 and 45, a proportion of earthworm compost produced by earthworms, in the form of a final nutrient medium resulting from a step of separating earthworms from their nutrient medium, **characterized in that** it comprises a mixture of different components, among which a major proportion by weight of brewers' grains, excluding the addition of earthworm compost.

2. The basic nutrient medium according to claim 1, **characterized in that** it comprises a mixture of a proportion of components selected from algae, legume hay, potatoes, buckwheat husks, spent mushroom substrate bales, straw and cardboard, in addition to brewers' grains.

3. The basic nutrient medium according to claim 2, **characterized in that** it comprises two, three or four of the components, selected from brewers' grains, algae, legume hay, potatoes, buckwheat husks, spent mushroom substrate bales, straw and cardboard.

4. The basic nutrient medium according to any one of claims 1 to 3, **characterized in that** it comprises brewers' grains, potatoes, buckwheat husks and spent mushroom substrate bales.

5. The basic nutrient medium according to any one of claims 1 to 4, **characterized in that** it comprises a mixture of different components reduced into small particles, the size of which is between 0 and 5 millimeters.

6. The basic nutrient medium according to any one of claims 1 to 5, **characterized in that** the proportion of earthworm compost that is added to the mixture of components is between 10 and 50% by weight.

7. A method for producing earthworms intended for animal feed, using the basic nutrient mixture according to any one of claims 1 to 6, which method consists in:
- producing a culture medium in the form of earthworm compost by earthworms,
- preparing a basic nutrient medium according to any one of claims 1 to 6,
- incorporating a quantity of earthworms into said basic nutrient medium,
- leaving the earthworms to develop during a determined growth period, while controlling the moisture level of the nutrient medium so that this moisture level is between 75 and 90%,
- at the end of the growth period, separating the earthworms from their nutrient medium,
- possibly implementing at least one earthworm transforming operation to obtain a transformed earthworm-based product,
- using earthworms, or the transformed earthworm-based product, for animal feed.

8. The method for producing earthworms according to claim 7, **characterized in that** it consists in leaving the earthworms to develop during a growth period of between two and four months.
